# EUROPÄISCHE PATENTANMELDUNG

(11) **EP 3 671 752 A1**
(43) Veröffentlichungstag der Anmeldung: **24.06.2020**
(21) Anmeldenummer: 18213077.3
(22) Anmeldetag: 17.12.2018
(51) Int. Cl.: G16H 20/30, G16H 40/63, A61H 5/00, A61F 9/00

(54) **TRAININGSSYSTEM ZUR STIMULATION DER AUGENBEWEGUNG SOWIE ZUM SELBSTSTÄNDIGEN TRAINIEREN DER AUGENBEWEGUNGSMUSKULATUR**

(71) Anmelder: Andersen, Sandra, 23611 Bad Schwartau (DE)
(72) Erfinder: Andersen, Eric, 23570 Lübeck-Travemünde (DE)
(74) Vertreter: Kailuweit & Uhlemann Patentanwälte Partnerschaft mbB

(57) **Zusammenfassung**

Die Erfindung betrifft ein Trainingssystem zur Stimulation der Augenbewegung sowie zum selbstständigen Trainieren der Augenbewegungsmuskulatur. Dabei weist das System elektrische Komponenten auf, welche voneinander beabstandet ausgebildet sind. Die elektrischen Komponenten umfassen mindestens eine Datenverarbeitungseinrichtung, mindestens eine Anzeigeeinrichtung zur optischen Signalisierung von veränderlich ausgebildeten Objekten, und mindestens einen Lautsprecher. Weiterhin weist das System ein am Kopf anordbar ausgebildetes Gehäuse und eine Haltevorrichtung, welche mit dem Gehäuse lösbar verbunden ist, auf. Dabei sind die elektrischen Komponenten zumindest teilweise im oder am Gehäuse angeordnet. Die Anzeigeeinrichtung ist vor den Augen eines Benutzers angeordnet und derart ausgebildet, mindestens ein Objekt in allen Raumrichtungen starr oder beweglich darzustellen, um das Blickfeld des Benutzers zu bedienen.

## Beschreibung

Die Erfindung betrifft ein Trainingssystem zur Stimulation der Augenbewegung sowie zum selbstständigen Trainieren der Augenbewegungsmuskulatur.

Vorrichtungen zum Trainieren der Augenbewegungsmuskulatur bzw. zur Behandlung von Fehlsichtigkeiten sind bereits aus einer Vielzahl von Dokumenten bekannt. Häufig verfolgen die Augen monokular oder binokular dabei sich bewegender Lichter oder blicken auf einen Gegenstand, der sich auf den Betrachter und von ihm wegbewegt. Dabei bleibt der Kopf häufig statisch und nur die Augen bewegen sich. Nachteilig an diesen Vorrichtungen ist kein gezieltes Training für eine gute Durchblutung, die Kräftigung, Dehnung und Entspannung der für die Bewegung der Augen zuständigen Muskeln möglich.

Die US 6,742,892 A beschreibt eine Vorrichtung zum Trainieren von Augenbewegungen, welche ein Gehäuse mit einer Vielzahl von Leuchtmitteln wie LEDs unterschiedlicher Farben zur Durchführung verschiedener Übungen umfasst, wobei die Leuchtmittel und deren Aktivierung über einen Controller gesteuert werden. Das Training der Augenbewegungen erfolgt dabei in horizontalen, vertikalen und schrägen Ebenen, nachteilig allerdings nur in einem begrenzten Bereich des Blickfeldes. Weiterhin wird durch die Aussendung verschiedener Wellenlängen die Fokussierkraft der Augen erhöht. Allerdings ist die Vorrichtung nicht tragbar und nicht geschlossen ausgebildet.

Die DE 20 2011 050 692 U1 offenbart eine als Brille oder Display ausgebildete Vorrichtung zur Lichtsimulation u.a. des Gewebes im Augenbereich mittels Leuchtmittel wie LEDs. Dabei kann die Frequenz der Helligkeitsänderung an das Ein- und Ausatmen des Benutzers gekoppelt sein. Zum Trainieren der Augenbewegungsmuskulatur werden die LEDs jeweils für eine bestimmte Zeit derart aktiviert, dass beide Augen einem Rhythmus entsprechend gerollt werden. Gleichzeitig beeinflussen diese Lichtimpulse die Netz- bzw. Aderhaut. Die Vorrichtung umfasst weiterhin auch eine Steuerelektronik sowie Mittel zum Abspielen von Audio-Signalen. Nachteilig wird durch die Vorrichtung nur eine Lichtstimulation ermöglicht.

In der WO 2012/166621 A1 wird ein Gerät zur Augenfunktionsmessung und zur Entspannung des Ziliarmuskels offenbart. Dabei umfasst das Gerät ein brillenartiges Gehäuse mit einem Kopfband, einer Lichtquelle, einen Lichtsensor und Ausrichtungslichtquellen wie LEDs. Dabei erfolgt die Trainingseinheit für die Bewegung der Augenmuskeln über eine Applikation auf einem mobilen Gerät wie beispielsweise einem Smartphone. Nachteilig ist es für den Benutzer nicht möglich, die durch die Lichtquellen dargestellten Lichtpunkte bis zum Rand seines Blickfeldes in einer Trainingseinheit zu trainieren, da das Gerät diesbezüglich nicht ausgebildet ist.

In der US 2005/0213034 A1 wird ein Augentrainingsgerät zur Verbesserung des Blickfeldes beschrieben, wobei das Gerät als Brille ausgebildet ist und mindestens zwei Irritationserzeugungsvorrichtungen aufweist, die im Bereich der Augen angeordnet sind und nacheinander betätigt werden. Indem es sich bei den Irritationserzeugungsvorrichtungen nicht um Lichtquellen, sondern andere Reiz-erzeugende Vorrichtungen wie Druck, Vibrationen oder Wärme handelt, wird ermöglicht, dass der Benutzer die Augenbewegungsmuskeln auch bei geschlossenen Augen bewegen kann, indem er lediglich in die Richtung blickt, aus welcher diese Irritationsreize kommen. Dabei können die Reize über das normal übliche Blickfeld hinausgehen und die Augäpfel über einen Blickwinkel von ca. 180° bewegt und trainiert werden. Über einen Kopfhörer werden dem Benutzer Anweisungen zur Ausführung der Übung oder entspannende Musik eingespielt. Nachteilig dient das Augentrainingsgerät lediglich zur Verbesserung des Blickfeldes, welches durch einen möglichen Gesichtsfeldausfall beeinträchtigt ist, aber nicht zur Verbesserung der Sehkraft.

Das Dokument US 2007/0200927 A1 beschreibt eine Vorrichtung zum Messen und Trainieren der Sehkraft, welche ein optisches Untersuchungssystem und eine damit gekoppelte Steuerung aufweist, und durch ein Band am Kopf des Benutzers befestigt wird. Durch Sehschlitze bzw. ein Display betrachtet der Benutzer eingespielte Bilder, wobei die Brennweite variabel einstellbar ist. Weiterhin werden dem Benutzer zusätzliche Reize durch am Untersuchungssystem angebrachte Lichter eingespielt. Dabei handelt es sich bei den eingespielten Bildern lediglich um optische Reproduktionen, umfassend Zeichnungen, Grafiken und Fotografien, welche weder softwaregesteuert, noch gezielt am oder bis zum Rand des Blickfeldes eingeblendet werden können. Durch die eingespielten Bilder ist es zwar möglich, zumindest den Ziliarmuskel zu trainieren, jedoch nicht alle relevanten Muskeln, welche für die Bewegung der Augen zuständig sind. Die Vorrichtung muss zudem nachteilig im Betrieb stets an einen extern befindlichen Computer angeschlossen sein.

Weiterhin existieren zahlreiche Geräte zur Realisierung von Lichttherapien mittels Lichtquellen, deren Prinzip u.a. auf der Stimulation der Augen über Licht mittels brillenartiger Vorrichtungen basieren. Dabei wird der Benutzer Licht mit variabler Intensität, Dauer, Bewegung und Farbe ausgesetzt. Allerdings verfolgen diese Therapien nicht den Zweck der Wiederherstellung der Sehkraft, sondern dienen eher der Entspannung und der Steigerung des Wohlbefindens. Als Beispiele wären hier u.a. die DE 103 01 867 A1, DE 295 14 342 U1 und EP 2 670 481 B1 zu nennen.

Die bisher veröffentlichten Dokumente verfolgen alle eine psychische Behandlung des Benutzers, indem u.a. die Augen in das Training einbezogen werden. Im Falle eines gezielten Trainings der Augenbewegungsmuskulatur werden nachteilig die Augen mit all den bekannten Geräten nicht bis an die Grenze des Blickfeldes geführt, sodass die Augenbewegungsmuskeln jeweils nur in einem beschränkten Bereich trainiert werden und somit ein effizientes und vollumfassendes Augenbewegungsmuskeltraining nicht gewährleistet werden kann.

Aufgabe ist es daher, ein Trainingssystem bereitzustellen, welches die Nachteile des Standes der Technik überwindet.

Durch das erfindungsgemäße System soll ein ganzheitliches Augenbewegungsmuskeltraining im Sinne einer Gymnastik der Augen bis hin zu den Grenzen des Blickfeldes gewährleistet werden, um so eine nicht-medizinische Verbesserung der Sehkraft und Kräftigung und Stärkung des Sehvermögens zu gewährleisten.

Weiterhin soll ein für den Benutzer motivierendes und belohnendes Trainingssystem zur Verfügung gestellt werden, welches einfach, selbstständig und komfortabel bedienbar ausgebildet und nicht auf medizinisches Personal wie beispielsweise Augenärzte beschränkt ist.

Erfindungsgemäß wird die Aufgabe durch ein Trainingssystem zur Stimulation der Augenbewegung sowie zum selbstständigen Trainieren der Augenbewegungsmuskulatur gemäß den unabhängigen Ansprüchen gelöst. Vorteilhafte Ausgestaltungen der Erfindung sind in den abhängigen Ansprüchen angegeben.

Ein erster Aspekt der Erfindung betrifft ein Trainingssystem zur Stimulation der Augenbewegung sowie zum selbstständigen Trainieren der Augenbewegungsmuskulatur, aufweisend elektrische Komponenten, welche voneinander beabstandet ausgebildet sind Dabei umfassen die elektrischen Komponenten mindestens eine Datenverarbeitungseinrichtung, mindestens eine Anzeigeeinrichtung zur optischen Signalisierung von veränderlich ausgebildeten Objekten und mindestens einen Lautsprecher, wobei die elektrischen Komponenten durch Mittel zur Signalübertragung lösbar miteinander verbunden sind. Weiterhin weist das Trainingssystem ein Gehäuse auf, welches am Kopf anordbar ausgebildet ist, und eine Haltevorrichtung, welche mit dem Gehäuse lösbar verbunden ist. Dabei sind die elektrischen Komponenten zumindest teilweise im oder am Gehäuse angeordnet sind. Die mindestens eine Anzeigeeinrichtung ist vor den Augen eines Benutzers angeordnet und derart ausgebildet, mindestens ein Objekt in allen Raumrichtungen starr oder beweglich darzustellen, um das Blickfeld des Benutzers zu bedienen. Dabei erfolgen die Augenbewegungen des Benutzers als Duktionen oder Torsionen.

Dabei entspricht das erfindungsgemäße Trainingssystem einem Trainingsgerät.

Vorteilhaft wird durch das erfindungsgemäße Trainingsgerät und dessen Anwendung am Benutzer eine Verbesserung der Sehkraft und des Sehvermögens erreicht, welche durch gezieltes Training der sechs Augenbewegungsmuskeln realisiert wird und mit einer Kräftigung, Dehnung, Entspannung, einem Aufbau und einer verbesserten Durchblutung dieser Augenbewegungsmuskeln einhergeht.

Durch das Training der Augenbewegungsmuskeln wird eine Kräftigung, Dehnung, Entspannung, ein Aufbau und eine verbesserte Durchblutung der Augenbewegungsmuskulatur ermöglicht. Dies erfolgt, indem die Augen Objekten folgen, welche visuell dargestellt werden, und je nach Bedarf durch eine akustische Anleitung dabei unterstützt werden.

Die Augenbewegungsmuskulatur eines Auges umfasst die äußeren Augenbewegungsmuskeln, welche für die Bewegung des Auges verantwortlich ist. Dabei wiederum weisen die äußeren Augenbewegungsmuskeln sechs Augenbewegungsmuskeln und deren dazugehörige Bewegungskomponenten auf. Im Sinne der Erfindung sind stets diese sechs äußeren Augenbewegungsmuskeln in Zusammenhang mit dem Training der Augenbewegungsmuskeln gemeint.

Durch das erfindungsgemäße Trainingssystem und die Verwendung des Trainingssystems in einem Verfahren wird die Augenbewegung stimuliert und dadurch die Augenbewegungsmuskulatur, insbesondere gezielt die sechs äußeren Augenbewegungsmuskeln, trainiert. Durch das Training wiederum wird die Augenbewegungsmuskulatur gekräftigt, gedehnt, entspannt und aufgebaut. Weiterhin erfolgt durch das Training eine verbesserte Durchblutung der Augenbewegungsmuskulatur.

Die sechs Augenbewegungsmuskeln bewegen das Auge in alle Richtungen und Orientierungen. Dabei sind die Bewegungskomponenten für die Hebung (Elevation), Senkung (Depression), Adduktion (d.h. Bewegung nach innen, zur Nase hin), Abduktion (d.h. Bewegung nach außen), Innenrollung und Außenrollung der sechs Augenbewegungsmuskeln zuständig.

Die sechs Augenbewegungsmuskeln der äußeren Augenbewegungsmuskeln weisen Quermuskeln und Längsmuskeln auf, wobei die Quermuskeln vier gerade Augenbewegungsmuskeln und die Längsmuskeln zwei schräge Augenbewegungsmuskeln aufweisen.

Die vier geraden Augenbewegungsmuskeln umfassen den musculus rectus superior, welcher das Auge nach oben und leicht nach innen zieht, den musculus rectus inferior, welches das Auge nach unten und leicht nach innen zieht, den muculus rectus lateralis, welcher das Auge nach außen zieht, sowie den musculus rectus medialis, welcher das Auge zur Mitte, d.h. in Richtung Nase zieht und der stärkste der vier geraden Augenbewegungsmuskeln ist.

Die zwei schrägen Augenbewegungsmuskeln ziehen das Auge von vorne medial, d.h. in Richtung Gesichtsmitte nach hinten lateral, d.h. in Richtung Außenseite. Die zwei schrägen Augenbewegungsmuskeln umfassen den musculus obliquus superior, welcher das Auge nach außen zieht und eine Drehung nach unten innen ermöglicht (Senkung mit Rollung des Auges nach innen, Inzykloduktion), sowie den musculus obliquus inferior, welcher das Auge nach außen zieht und eine Drehung nach oben außen ermöglicht (Rollung des Auges nach außen, Exyzkloduktion, sowie Hebung in Adduktion).

Es ist bekannt, dass der Akkommodationsprozess, also die Fähigkeit des Auges, die Brechkraft dynamisch anzupassen, von den Augenbewegungsmuskeln ausgelöst wird. Insbesondere tragen die Augenbewegungsmuskeln des Auges, basierend auf der Sehkrafttheorie nach William Bates, zur Akkommodation des Auges bei.

Sind alle Augenbewegungsmuskeln entspannt, hat das Auge die Form einer Kugel. Dabei befindet sich der Brennpunkt (Fokus) direkt auf der Netzhaut und das entspannte Auge ist in der Lage, scharf in der Ferne zu sehen. Soll hingegen in der Nähe scharf gesehen werden, muss der Brennpunkt tiefer im Auge liegen. Dabei werden die Längsmuskeln entspannt und die Quermuskeln angespannt. Indem das Auge zusammengedrückt wird, verlängert sich das Auge nach vorne, der Fokus liegt somit tiefer im Auge und ein so gewölbtes Auge sieht dadurch gut in der Nähe. Dies ist die Grundlage der wissenschaftlichen Arbeit über die Sehkrafttheorie und des damit einhergehenden Augentrainings von und nach William Bates (vgl. z.B. Rechtes Sehen ohne Brille: Heilung fehlerhaften Sehens durch Behandlung ohne Brille, von Oswald Roth (Herausgeber), William H Bates (Autor), Elsbeth Friedrichs (Übersetzer), Rohm-Verlag, 5. Auflage, Oktober 2007).

Das Augentraining nach Bates ist nicht-medizinisch ausgerichtet und steht in keinem Zusammenhang mit den klassischen Sehschulen, welche diagnostische und therapeutische Leistungen erbringen. Daher wird das Augentraining nach Bates auch nicht von medizinisch ausgebildetem Fachpersonal angewandt. Das erfindungsgemäße Trainingssystem und das damit einhergehende Trainieren durch die Verwendung des Trainingssystems in einem Verfahren zur Stimulation der Augenbewegung, einschließlich der Durchblutung der sechs Augenbewegungsmuskeln, basiert auf den Erkenntnissen der Sehkrafttheorie nach Bates.

Unter Sehkraft wird im Sinne der Erfindung die Sehstärke und somit das Auflösungsvermögen des Auges verstanden.

Bei einer oder mehreren auftretenden Fehlfunktion dieser sechs Augenbewegungsmuskeln, d.h. wenn diese entweder einzeln nicht richtig funktionieren oder im Zusammenspiel nicht richtig aufeinander abgestimmt sind, kommt es zu Sehkraftstörungen. In Ausführungsformen umfassen diese Sehkraftstörungen bzw. Störungen des Sehvermögens vier fehlerhafte Ausprägungen in der Anatomie des Auges, nämlich Kurzsichtigkeit, Weitsichtigkeit, Schielen und Astigmatismus.

Durch spezielle Übungen, welche in Form von Trainingseinheiten eines Trainingsplans durch das erfindungsgemäße Trainingssystem realisiert werden, kann die normale Arbeit der Augenbewegungsmuskeln ganzheitlich wiederhergestellt und Fehlfunktionen der sechs Augenbewegungsmuskeln behoben werden, indem geschwächte Augenbewegungsmuskeln automatisch mittrainiert werden. Und in Folge davon können Kurzsichtigkeit, Weitsichtigkeit, Schielen und Astigmatismus verschwinden bzw. deren Auswirkungen eingegrenzt werden.

Erfindungsgemäß weist das Trainingssystem elektrische Komponenten auf. Im Sinne der Erfindung sind die elektrischen Komponenten voneinander beabstandet ausgebildet.

In einer Ausführungsform sind die elektrischen Komponenten je nach Einsatzzweck und Bedarf auswählbar. In einer weiteren Ausführungsform sind die elektrischen Komponenten austauschbar ausgebildet.

Erfindungsgemäß sind die elektrischen Komponenten durch Mittel zur Signalübertragung lösbar miteinander verbunden. Durch die Mittel zur Signalübertragung erfolgt eine informationstechnische Verbindung der elektrischen Komponenten untereinander. In einer Ausführungsform sind die Mittel zur Signalübertragung als drahtgestützte oder drahtlose Verbindung ausgebildet.

In einer Ausführungsform, im Falle einer drahtgestützten Verbindung, sind die Mittel zur Signalübertragung zumindest teilweise als elektrisches Kabel ausgebildet. In einer weiteren Ausführungsform, im Falle einer drahtlosen Verbindung, sind die Mittel zur Signalübertragung zumindest teilweise als Bluetooth- oder WLAN-Verbindung ausgebildet.

Erfindungsgemäß umfassen die elektrischen Komponenten mindestens eine Datenverarbeitungseinrichtung. Die Datenverarbeitungseinrichtung wird auch als Signalverarbeitungsplattform bezeichnet.
In Ausführungsformen ist die mindestens eine Datenverarbeitungseinrichtung im oder am Gehäuse des erfindungsgemäßen Trainingssystems angeordnet. In Ausführungsformen ist die mindestens eine Datenverarbeitungseinrichtung als Mikroprozessor ausgebildet. In Ausführungsformen wird durch die Datenverarbeitungseinrichtung zumindest eine Teilkomponente des Computerprogrammprodukts und somit der auf dem Computerprogrammprodukt aufgespielte Trainingsplan angesteuert.

In Ausführungsformen ist die Datenverarbeitungseinrichtung derart ausgebildet, um ein szenarioabhängiges Steuersignal über Mittel zur Signalübertragung an die mindestens eine Anzeigeeinrichtung und/oder den mindestens einen Lautsprecher zu übermitteln.

In einer bevorzugten Ausführungsform ist die mindestens eine Datenverarbeitungseinrichtung extern von dem erfindungsgemäßen Trainingssystem angeordnet. Dabei bedeutet "extern" im Sinne der Erfindung, dass die Datenverarbeitungseinrichtung räumlich außerhalb des Trainingssystems angeordnet ist und somit beispielsweise nicht im oder am Gehäuse. Vorteilhaft werden damit das Gewicht und die Kompaktheit des Gehäuses reduziert.

In einer bevorzugten Ausführungsform ist die mindestens eine Datenverarbeitungseinrichtung über ein Modul zur Datenübertragung mit einem externen Ausgabegerät gekoppelt.

Erfindungsgemäß umfassen die elektrischen Komponenten mindestens eine Anzeigeeinrichtung zur optischen Signalisierung von veränderlich ausgebildeten Objekten.

In Ausführungsformen ist die Anzeigeeinrichtung im oder am Gehäuse des erfindungsgemäßen Trainingssystems angeordnet.

Erfindungsgemäß ist die mindestens eine Anzeigeeinrichtung vor den Augen eines Benutzers angeordnet und derart ausgebildet, mindestens ein Objekt in allen Raumrichtungen starr oder beweglich darzustellen, um das maximale Blickfeld des Benutzers zu bedienen. In Ausführungsformen gibt die mindestens eine Anzeigeeinrichtung eine zweidimensionale Ansicht der Objekte wieder. Vorteilhaft ist die Anzeigeeinrichtung so im Gehäuse angeordnet, dass die Anzeigeeinrichtung im jeweiligen Blickfeld des Benutzers liegt.

In Ausführungsformen ist das erfindungsgemäße Trainingssystem und das damit einhergehende Training für das binokulare Training, also beide Augen betreffend, oder für das monokulare Training, als nur ein Auge betreffend, ausgebildet. Im Folgenden sind unter der Formulierung "Auge", je nach gewünschter Behandlung, stets das linke Auge oder das rechte Auge oder beide Augen inbegriffen. Bei einer binokularen Anwendung des erfindungsgemäßen Trainingssystems besteht das Bedürfnis, beide Augen zu trainieren und zu kräftigen, bei einer monokularen Anwendung des erfindungsgemäßen Trainingssystems hingegen weist nur ein Auge Verbesserungspotential auf.

Im Sinne der Erfindung wird dabei unter "Raumrichtung" jeweils eine durch die Objekte imaginäre aufgespannte Ebene innerhalb der Anzeigeeinrichtung verstanden, wobei die Ebene jeweils eine horizontale, vertikale oder diagonale Ausrichtung innerhalb der zweidimensionalen Bildfläche der Anzeigeeinrichtung einnimmt. Dabei bezieht sich die horizontale Ausrichtung der Ebene auf die Bewegung der Objekte von links nach rechts und die vertikale Ausrichtung der Ebene auf die Bewegung der Objekte von oben nach unten, und jeweils umgekehrt. Weiterhin bezieht sich die diagonale Ausrichtung der Ebene auf die Bewegung der Objekte schräg über die Bildfläche. Dabei können die Objekte innerhalb dieser generierten zweidimensionalen Bildfläche in beliebiger Kombination hinsichtlich der zeitlichen und örtlich-geometrischen Abfolge sowie der Intensität in den Raumrichtungen erscheinen.

Weiterhin wird unter einer "beweglichen Darstellung" eine sich in zeitlicher und geometrischörtlicher Abfolge bzw. in variablen Abfolgen erscheinende Darstellung der Objekte verstanden. So kann beispielsweise die Taktlänge der erscheinenden Objekte variiert und individuell angepasst werden. Die Abfolge der Objekte wiederum ist im Trainingsplan auf dem Computerprogrammprodukt enthalten.

In weiteren bevorzugten Ausführungsformen sind die Objekte auf der mindestens einen Anzeigeeinrichtung in einer variablen Intensität darstellbar, wobei sich die Intensität auf Licht- und Farbintensität bezieht. Weiterhin weist die mindestens eine Anzeigeeinrichtung eine Einblend- und Ausblendfunktion der Objekte auf.

Im Sinne der Erfindung wird unter dem "Blickfeld" des Benutzers alle mit den Augen fixier- und fokussierbaren Objekte verstanden, wenn der Körper und der Kopf des Benutzers statisch, starr bzw. ruhig gehalten werden und sich nicht bewegen oder drehen, sodass sich nur das oder die Augen des Benutzers bewegen. In Ausführungsformen der Erfindung umfasst der Begriff des Blickfelds sowohl das monokulare Blickfeld als auch das binokulare Blickfeld. Das Blickfeld unterscheidet sich vom Gesichtsfeld, welches bei ruhiger Kopfhaltung und geradeaus gerichtetem, bewegungslosen Blick vorliegt und zudem auf subjektiven sensitiven Wahrnehmungsempfindlichkeiten beruht.

Dabei entspricht das maximale Blickfeld hinsichtlich der ergonomischen Qualität jeweiligen äußeren Grenzen des Blickfeldes des Benutzers in allen Raumrichtungen und Dimensionen und folglich der vollständigen Auslastung des Blickfeldes. Dabei bezieht sich die horizontale Richtung auf die Bewegung der Augen von links nach rechts und umgekehrt. Weiterhin bezieht sich die vertikale Richtung auf die Bewegung der Augen von oben nach unten und umgekehrt. Vorteilhaft wird durch das erfindungsgemäße Trainingssystem und dessen Verwendung in einem Verfahren zur Stimulation der Augenbewegung und zum selbstständigen Trainieren das maximale Blickfeld des Benutzers trainiert.

Vorteilhaft werden durch das erfindungsgemäße Trainingssystem und dessen Verwendung in einem Verfahren zur Stimulation der Augenbewegung und zum selbstständigen Trainieren und der damit verbundenen Ausnutzung des maximalen Blickfelds die Augenbewegungsmuskeln gleichmäßig und in einem großen Bereich, selbst für Personen mit eingeschränktem Blickfeld, bis an die jeweils maximalen Grenzen des Blickfelds, trainiert.

In einer bevorzugten Ausführungsform ist die mindestens eine Anzeigeeinrichtung als Display ausgebildet. Dabei kann die Anzeigeeinrichtung auch miniaturisiert ausgebildet sein, beispielsweise als Mikrodisplay. Das Display entspricht weiterhin auch einem Bildschirm oder Monitor. In Ausführungsformen umfassen die Darstellungstechniken der mindestens einen Anzeigeeinrichtung einen Plasmabildschirm, einen LCD-Bildschirm (Flüssigkristallbildschirm), Leuchtdioden (LEDs) oder organische Leuchtdioden (OLEDs). In alternativen Ausführungsformen ist die mindestens eine Anzeigeeinrichtung als 3D-Monitor bzw. Stereodisplay ausgebildet und vermittelt eine dreidimensionale Ansicht der Objekte für einen Tiefeneindruck durch stereoskopisches Sehen.

In Ausführungsformen ist die mindestens eine Anzeigeeinrichtung in Richtung Benutzer gekrümmt ausgebildet, um eine Panoramasicht bei den einzelnen Trainingseinheiten zu gewährleisten. In weiteren Ausführungsformen ist die mindestens eine Anzeigeeinrichtung flach ausgebildet.

In weiteren Ausführungsformen kann es sich bei der Anzeigeeinrichtung um kommerziell erhältliche Anzeigeeinrichtungen handeln. Vorteilhaft wird dem Benutzer durch die Anzeigeeinrichtung eine visuelle Trainingsanleitung bereitgestellt.

In einer Ausführungsform sind die Objekte und/oder die restliche Bildfläche der Anzeigeeinrichtung, welche keine Objekte zeigt, aktiv leuchtend und/oder nicht leuchtend ausgebildet.

In Ausführungsformen weisen die elektrischen Komponenten mehrere Anzeigeeinrichtungen auf, welche über die gesamte Fläche des Gehäuses verteilt sein können. Dabei ist zumindest eine Anzeigeeinrichtung im Blickfeld des Benutzers angeordnet.

In einer Ausführungsform werden die Objekte über die mindestens eine Anzeigeeinrichtung eingespielt. Dabei folgt der Benutzer den eingespielten Objekten mit einem Auge oder mit beiden Augen und fokussiert dabei gleichzeitig auf die Objekte. In Ausführungsformen erfolgt die Bewegung der Objekte in allen Raumrichtungen, welche je nach Trainingsplan ausgewählt werden. Dabei erfolgen die Augenbewegungen des Benutzers bevorzugt als Duktionen oder Torsionen. Beispielsweise führen die Augen (oder das Auge) eine rollende Bewegung, beispielsweise im Uhrzeigersinn, oder eine Hin- und Herbewegung aus.

In einer Ausführungsform sind die veränderlich ausgebildeten Objekte als abstrakte Punkte, Striche, Linien, Kurven oder Felder oder einer Kombination aus diesen Objekten ausgebildet. Da diese Objekte von selbst leuchten, werden sie auch als Lichtpunkte, Lichtstriche, Lichtlinien, Lichtkurven oder Lichtfelder bezeichnet. In einer weiteren Ausführungsform sind die veränderlich ausgebildeten Objekte als konkrete Figuren oder Buchstaben ausgebildet. Dabei können die Objekte zwischen diesen Ausgestaltungen beliebig wechseln. Die Objekte werden dabei bis zu den Grenzen des jeweiligen Blickfeldes des Benutzers dargestellt, wodurch das jeweilige maximale Blickfeld des Benutzers trainiert wird.

In Ausführungsformen werden die Objekte durch die Anzeigeeinrichtung festgelegt und realisiert. Dabei ist die Anzeigeeinrichtung informationstechnisch mit dem Computerprogrammprodukt verbunden, welches die visuelle Trainingsanleitung in Form des Trainingsplans enthält.

Vorteilhaft fokussiert der Benutzer ein Auge oder beide Augen auf die eingespielten Objekte.

Erfindungsgemäß umfassen die elektrischen Komponenten mindestens einen Lautsprecher. Dabei entspricht der mindestens eine Lautsprecher einer Ausgabeeinrichtung und dient mindestens dem Empfang eines Monosignals. In Ausführungsform ist der mindestens eine Lautsprecher vor mindestens einem Ohr des Benutzers oder in Hörweite angeordnet. In Ausführungsformen weist das Gehäuse hierzu einen Lautsprecheranschluss auf.

In einer Ausführungsform ist der mindestens eine Lautsprecher als Kopfhörer ausgebildet. Dazu weist das Gehäuse einen Kopfhöreranschluss auf. Vorteilhaft liegt der Kopfhörer derart an den Ohren des Benutzers an, dass dieser ein stereophones Signal empfängt. In Ausführungsformen ist der Kopfhörer als In-Ear-Kopfhörer oder Stereo-Kopfhörer ausgebildet. In Ausführungsformen kann es sich bei dem Lautsprecher um einen kommerziell erhältlichen Lautsprecher, insbesondere einen kommerziell erhältlichen Kopfhörer unterschiedlicher Ausführungsformen handeln.

In Ausführungsformen ist der mindestens eine Lautsprecher intern im oder am Gehäuse des erfindungsgemäßen Trainingssystems angeordnet.

Vorteilhaft wird durch den mindestens einen Lautsprecher neben den visuellen Reizen auf das Auge oder die Augen eine zusätzliche akustische Verstärkung und Intensivierung des Trainings gegeben, indem der Benutzer weitere Reize, beispielsweise in Form von Anweisungen oder Empfehlungen, über den Lautsprecher empfängt.

Dabei ist der Lautsprecher informationstechnisch mit dem Computerprogrammprodukt verbunden, welches die akustische Trainingsanleitung in Form des Trainingsplans enthält.

Erfindungsgemäß weist das Trainingssystem ein Gehäuse auf, welches am Kopf des Benutzers anordbar ausgebildet ist.

In einer bevorzugten Ausführungsform ist das Gehäuse als Brille oder Maske ausgebildet. In Ausführungsformen kann es sich bei dem Gehäuse um ein kommerziell erhältliches Gehäuse, insbesondere eine kommerziell erhältliche Brille unterschiedlicher Ausführungsformen handeln. In einer alternativen Ausführungsform ist das Gehäuse als VR-Brille ausgebildet.

In Ausführungsformen ist das Gehäuse flexibel an die jeweilige Anatomie des das Trainingssystem tragenden Benutzers einstellbar und anpassbar ausgebildet. Dabei bezieht sich die flexible Einstellbarkeit und Anpassbarkeit des Gehäuses u.a. auf die Kopfgröße oder die Größe des Gesichts eines Kindes oder eines Erwachsenen. Vorteilhaft wird somit der Tragekomfort erhöht.

In einer Ausführungsform ist das Gehäuse zumindest teilweise gepolstert ausgebildet, insbesondere jenem Bereich, welcher in Kontakt mit dem Gesicht des Benutzers ist, wodurch vorteilhaft der Tragekomfort für den Benutzer gesteigert wird.

Vorteilhaft ist das Gehäuse tragbar ausgebildet.

In Ausführungsformen ist das Gehäuse im erfindungsgemäßen Trainingssystem angeordnet.

Erfindungsgemäß sind die elektrischen Komponenten zumindest teilweise im oder am Gehäuse angeordnet.

Erfindungsgemäß weist das Trainingssystem eine Haltevorrichtung auf, welche mit dem Gehäuse lösbar verbunden ist. In Ausführungsformen ist die Haltevorrichtung im erfindungsgemäßen Trainingssystem angeordnet, bevorzugt am Gehäuse des erfindungsgemäßen Trainingssystems.

Vorteilhaft dient die Haltevorrichtung dazu, dass das Gehäuse des Trainingssystems am Kopf des Benutzers tragbar ist.

In einer bevorzugten Ausführungsform ist die Haltevorrichtung als verstellbares Band, Gurtband oder Gurt ausgebildet. In einer weiteren Ausführungsform weist die Haltevorrichtung Verschlussmittel, insbesondere mechanische Verschlussmittel auf. Dabei umfassen die mechanischen Verschlussmittel Schnallen, insbesondere Rollschnallen, Doppelsteg-Schnallen, Dreisteg-Schnallen, Leiterschnallen, Schiebeschnallen, Klemmschnallen, Klickschnallen (Blitz- oder Klickverschluss, auch Klickschließen genannt), Steckschnallen oder Formschlussschnallen. Weiterhin umfassen die mechanischen Verschlussmittel Knebelverschlüsse, Klettverschlüsse, Druckknöpfe, Reißverschlüsse oder Steckschlösser.

In Ausführungsformen ist die Haltevorrichtung flexibel an die jeweilige Anatomie des das Trainingssystem tragenden Benutzers einstellbar und anpassbar ausgebildet. Dabei bezieht sich die flexible Einstellbarkeit und Anpassbarkeit der Haltevorrichtung u.a. auf die Kopfgröße oder die Größe des Gesichts eines Kindes oder eines Erwachsenen.

In einer bevorzugten Ausführungsform umfasst das erfindungsgemäße Trainingssystem weiterhin mindestens ein Fokussierrad, welches am Gehäuse angeordnet ist. Vorteilhaft wird durch das mindestens eine Fokussierrad eine manuelle Einstellung der Sehschärfe des mindestens einen trainierten Auges durch den Benutzer oder den Behandler ermöglicht. Dabei kann die Einstellung der Sehschärfe manuell erfolgen.

In einer bevorzugten Ausführungsform umfasst das erfindungsgemäße Trainingssystem mindestens einen Energiespeicher. Dabei dient der Energiespeicher der Energieversorgung des erfindungsgemäßen Trainingssystems.

In einer Ausführungsform ist der Energiespeicher als Ladegerät, insbesondere als Batterie oder Akkumulator ausgebildet und kann beispielsweise über einen USB- oder Klinkenstecker wieder aufgeladen werden.

In Ausführungsformen ist der mindestens eine Energiespeicher im oder am Gehäuse des erfindungsgemäßen Trainingssystems angeordnet. In einer bevorzugten Ausführungsform ist der mindestens eine Energiespeicher extern angeordnet. Dabei bedeutet "extern" im Sinne der Erfindung, dass der Energiespeicher räumlich außerhalb des Trainingssystems angeordnet ist und somit beispielsweise nicht im oder am Gehäuse. Vorteilhaft werden damit das Gewicht und die Kompaktheit des Gehäuses reduziert.

Ein weiterer Aspekt der Erfindung betrifft ein Trainingssystem zur Verwendung in einem Verfahren zur Stimulation der Augenbewegung sowie zum selbstständigen Trainieren der Augenbewegungsmuskulatur.

Erfindungsgemäß umfasst das Verfahren das Anordnen des Gehäuses durch die Haltevorrichtung am Kopf des Benutzers, sodass die mindestens eine Anzeigeeinrichtung vor den Augen und der mindestens eine Lautsprecher vor mindestens einem Ohr des Benutzers angeordnet ist. Weiterhin umfasst das erfindungsgemäße Verfahren anschließend die Darstellung von Objekten in allen Raumrichtungen, auf welche sich der Benutzer fokussiert und dabei mindestens ein Auge am Rande des Blickfelds bewegt, und die akustische Wiedergabe von Geräuschen, auf welche der Benutzer reagiert. Bei der Darstellung von Objekten in allen Raumrichtungen werden die Objekte durch die Augen fixiert und fokussiert und die Augen folgen deren Bewegungen. Empfehlenswert ist, dass der Benutzer zum Training eine ruhige Körperposition einnimmt und Körper und/oder Kopf nur geringfügig bewegt oder dreht. So kann der Benutzer sich ganz auf das Training konzentrieren. Folglich findet auch keine Kopfbewegung während des Trainings statt. Andererseits kann das erfindungsgemäße Trainingssystem und dessen Verwendung im Verfahren auch dann verwendet werden, wenn der Benutzer Kopf und/oder Körper währenddessen bewegt - ein ruhiges Verharren ist für das Training nicht zwingend notwendig. Im Sinne der Erfindung wird durch die Datenverarbeitungseinrichtung und/oder durch das externe Ausgabegerät ein szenarioabhängiges Steuersignal über die Mittel zur Signalübertragung an die mindestens eine Anzeigeeinrichtung und/oder den mindestens einen Lautsprecher übermittelt, wodurch der Trainingsablauf vorgegeben oder selbstständig angepasst wird.

Durch das szenarioabhängige Steuersignal wird zumindest ein Teil der elektrischen Komponenten des erfindungsgemäßen Trainingssystems aktiviert.

In einer bevorzugten Ausführungsform umfasst das Verfahren zur Stimulation der Augenbewegung sowie zum selbstständigen Trainieren der Augenbewegungsmuskulatur weiterhin, dass über mindestens einen Sensor die Bewegung der Augen des Benutzers als Daten erfasst werden und die erfassten Daten über Mittel zur Signalübertragung an die mindestens eine Datenverarbeitungseinrichtung übermittelt werden, und die Daten von der Datenverarbeitungseinrichtung an ein externes Ausgabegerät übermittelt werden. Im Sinne der Erfindung werden die Daten durch die mindestens eine Datenverarbeitungseinrichtung und/oder das externe Ausgabegerät verarbeitet und ausgewertet und darauf basierend ein szenarioabhängiges Steuersignal über die Mittel zur Signalübertragung an die mindestens eine Anzeigeeinrichtung und/oder den mindestens einen Lautsprecher übermittelt, wodurch das Training selbstständig angepasst wird.

In einer Ausführungsform kann die Trainingsdauer je nach den Erfordernissen des Benutzers angepasst werden. Typischerweise wird eine Trainingsdauer 3mal täglich (früh, mittags, abends), bevorzugt vor den Mahlzeiten in einem Zeitraum von jeweils 5-20 min realisiert.

Erfindungsgemäß umfassen die elektrischen Komponenten des Trainingssystems mindestens ein externes Ausgabegerät.

In einer bevorzugten Ausführungsform ist das externe Ausgabegerät so gestaltet, dass es ein szenarioabhängiges Steuersignal über Mittel zur Signalübertragung an die Datenverarbeitungseinrichtung sendet, welches wiederum an die mindestens eine Anzeigeeinrichtung und/oder den mindestens einen Lautsprecher übermitteltwird.

In Ausführungsformen wird durch das externe Ausgabegerät zumindest eine Teilkomponente des Computerprogrammprodukts und somit der auf dem Computerprogrammprodukt aufgespielte Trainingsplan angesteuert.

In einer Ausführungsform ist das externe Ausgabegerät als Computer, Tablet, Laptop oder Smartphone gestaltet.

In einer bevorzugten Ausführungsform weist das erfindungsgemäße Trainingssystem ein Computerprogrammprodukt auf. Das Computerprogrammprodukt und die elektrischen Komponenten bilden das Computersystem des erfindungsgemäßen Trainingssystems.

In einer weiteren bevorzugten Ausführungsform ist das Computerprogrammprodukt auf der Datenverarbeitungseinrichtung und/oder dem externen Ausgabegerät gespeichert.

Bevorzugt weist das Computerprogrammprodukt eine erste Teilkomponente auf, welche als visuelle Trainingsanleitung dient. In Ausführungsformen ist die visuelle Trainingsanleitung als Anweisung wie beispielsweise einer in Worten dargestellten Anleitung, auf der Anzeigeeinrichtung und somit für den Benutzer sichtbar ausgebildet. Vorteilhaft wird dem Benutzer durch die Anzeigeeinrichtung eine visuelle Trainingsanleitung bereitgestellt. Vorteilhaft wird dadurch ein durchgängig motivierendes Training für den Benutzer gewährleistet.

In einer bevorzugten Ausführungsform ist die erste Teilkomponente des Computerprogrammprodukts auf der Datenverarbeitungseinrichtung und/oder dem externen Ausgabegerät gespeichert.

In einer weiteren bevorzugten Ausführungsform weist das Computerprogrammprodukt eine zweite Teilkomponente auf, welche als akustische Trainingsanleitung dient. In Ausführungsformen ist die akustische Trainingsanleitung als Personaltrainer, Audioguide oder Sprachführer ausgebildet. Vorteilhaft wird dem Benutzer durch den Lautsprecher eine akustische Trainingsanleitung bereitgestellt. Vorteilhaft wird dadurch, in Kombination mit der visuellen Trainingsanleitung, ein durchgängig motivierendes Training für den Benutzer gewährleistet.

In weiteren bevorzugten Ausführungsformen sind die erste und die zweite Teilkomponente des Computerprogrammprodukts miteinander informationstechnisch gekoppelt.

In einer weiteren bevorzugten Ausführungsform ist die zweite Teilkomponente des Computerprogrammprodukts auf der Datenverarbeitungseinrichtung und/oder dem externen Ausgabegerät gespeichert.

In einer Ausführungsform realisiert das Computerprogrammprodukt mindestens einen Trainingsplan. Der Trainingsplan stellt das Trainingsprogramm für den Benutzer dar.

Vorteilhaft erfolgt eine benutzerfreundliche Bedienung der Datenverarbeitungseinrichtung oder des externen Ausgabegerätes durch das Computerprogrammprodukt. Beispielsweise werden die Daten durch die Datenverarbeitungseinrichtung oder das externe Ausgabegerät für den Benutzer visualisiert.

In Ausführungsformen ist das Computerprogrammprodukt durch regelmäßige Updates aktualisierbar. Vorteilhaft kann der Benutzer dadurch weitere Trainingspläne auf das erfindungsgemäße Trainingssystem aufspielen. In weiteren Ausführungsformen ist das Computerprogrammprodukt als App ausgebildet und kann regelmäßig aktualisiert werden. Dazu weist die Datenverarbeitungseinrichtung oder das externe Ausgabegerät eine Schnittstelle auf, welche beispielsweise einen Internetzugang ermöglicht.

In einer Ausführungsform ist der Trainingsplan für eine elementare Augengymnastik individuell auf die Bedürfnisse des jeweiligen Benutzers anpassbar ausgebildet. Dabei bezieht sich "individuell" in Abhängigkeit der Dauer und Stärke des Augenbewegungsmuskeltrainings. Dabei ist der Trainingsplan vorteilhaft abwechslungsreich, motivierend und leistungsfördernd ausgebildet. So können beispielsweise Smileys oder andere lobende Abbildungen bei erfolgreicher Absolvierung einer Trainingseinheit oder eines Schwierigkeitsgrades über die Anzeigeeinrichtung eingeblendet werden. Ebenso ist es möglich, dass sprachliches Lob durch den Lautsprecher übermittelt wird. Der Trainingsplan umfasst die akustische und/oder visuelle Trainingsanleitung und ist für den Benutzer visuell und/oder akustisch über das Computerprogrammprodukt, insbesondere über die erste und/oder zweite Teilkomponente des Computerprogrammprodukts, abrufbar.

Weiterhin sollen die Trainingseinheiten verstellbar ausgebildet sen, indem die Darstellung der Objekte auf der Anzeigeeinrichtung in veränderlich anpassbaren Stufen im Blickfeld des Benutzers eingespielt werden. Dabei werden die Objekte in einem sich nach und nach größer werdenden Bereich des Blickfeldes bis hin zum maximalen Blickfeld eingespielt. Das sich dynamisch bis hin zum maximalen Blickfeld skalierende und vergrößernde Blickfeld kann innerhalb einer Trainingseinheit, oder über mehrere Trainingseinheiten verteilt, vorgenommen werden. Die dynamische Skalierung des Blickfeldes und der darin angezeigten Objekte erfolgt durch die Trainingseinheiten. Durch diese flexible und individuell anpassbare graduelle allmähliche Erweiterung des Blickfeldes können vorteilhaft auch Benutzer mit eingeschränktem Blickfeld das Trainingsprogramm absolvieren und das Training nach und nach an ein größeres Blickfeld anpassen, ohne dass es durch eine am Anfang volle Ausreizung des maximalen Blickfeldes zu Überforderungen kommt. In einer alternativen Ausführungsform kann das Blickfeld auch vom maximalen Blickfeld ausgehend, verkleinert werden.

In Ausführungsformen weist der Trainingsplan mindestens einen Schwierigkeitsgrad auf. Die Schwierigkeitsgrade wiederum beinhalten mindestens eine Trainingseinheit, bevorzugt mehrere Trainingseinheiten. Per Update können weitere Trainingseinheiten oder Schwierigkeitsgrade zum Trainingsplan hinzugefügt werden.

In Ausführungsformen umfasst jede einzelne Trainingseinheit das Anzeigen von Objekten in verschieden ausgebildeten Taktlängen, welche in Abhängigkeit von der Trainingseinheit variierbar ausgebildet sind. In einer Ausführungsform weist eine Taktlänge eine Dauer im Bereich von einer bis mehrere Sekunden auf. In Ausführungsformen liegt die Dauer einer Taktlänge im Bereich von einer Sekunde bis zu 10 Sekunden.

In einer Ausführungsform wird durch die erfindungsgemäße Trainingsvorrichtung ein monokulares oder ein binokulares Training des oder der Augen des Benutzers realisiert.

In Ausführungsformen ist das erfindungsgemäße Trainingssystem tragbar ausgebildet. Vorteilhaft kann der Benutzer das System somit mobil mit sich führen und an allen möglichen Orten und jederzeit je nach Bedarf benutzen.

In einer Ausführungsform ist das Gehäuse des erfindungsgemäßen Trainingssystems mit den darin angeordneten elektrischen Komponenten individuell an den Benutzer angepasst. Dabei weist dieses personalisiert ausgebildete Gehäuse einen Sensor auf, welcher nur dem berechtigten Benutzer die Verwendung erlaubt. So kann der Sensor beispielsweise als Fingerabdrucksensor ausgebildet sein. In einer alternativen Ausführungsform ist das Gehäuse des erfindungsgemäßen Trainingssystems mit den darin angeordneten elektrischen Komponenten für mehrere Benutzer verwendbar ausgebildet. Vorteilhaft können so zum Beispiel mehrere Familienmitglieder das Gehäuse nutzen.

Das erfindungsgemäße Trainingssystem ist insbesondere für medizinisch nicht vorgebildete Benutzer konstruiert und kann somit vorteilhaft von jedem Benutzer ohne medizinische Vorkenntnisse verwendet werden. Da es sich bei dem erfindungsgemäßen Trainingssystem um kein Medizinprodukt handelt, erfolgt eine nicht-medizinische Stimulation der Augenbewegung sowie ein nicht-medizinisches selbstständigen Trainieren der Augenbewegungsmuskulatur. Das Training entspricht daher einer Augengymnastik, welche jederzeit selbstständig durch den Benutzer, ohne das Heranziehen oder der Betreuung eines Arztes oder medizinischen Fachpersonals, selbst bei eingeschränktem Blickfeld des Benutzers, durchgeführt werden kann. Durch diese Augengymnastik wird die Augenmuskulatur gekräftigt, gedehnt, entspannt und aufgebaut. Weiterhin dient das erfindungsgemäße Trainingssystem einer verbesserten Durchblutung der Augenbewegungsmuskeln. Bei regelmäßiger Anwendung des erfindungsgemäßen Trainingssystems wird die Sehkraft und das Sehvermögen des Benutzers erheblich verbessert.

Das erfindungsgemäße Trainingssystem kann weiterhin sowohl von Erwachsenen als auch von Kindern benutzt werden. Vorteilhaft kann durch die anatomische Anpassbarkeit von Gehäuse bzw. Haltevorrichtung das Trainingssystem für mehrere Benutzer, beispielsweise innerhalb einer Familie, verwendet werden.

In einer bevorzugten Ausführungsform wird das erfindungsgemäße Trainingssystem zur Stimulation der Augenbewegung sowie zum selbstständigen Trainieren der Augenbewegungsmuskulatur verwendet. Vorteilhaft werden dadurch, je nach Anwendung auf einem oder beiden Augen, die sechs Augenbewegungsmuskeln gekräftigt.

In einer weiteren bevorzugten Ausführungsform wird das erfindungsgemäße Trainingssystem zur Verbesserung der Sehkraft oder zur Kräftigung und Stärkung des Sehvermögens durch das gezielte Trainieren der sechs Augenbewegungsmuskeln verwendet.

Die Verbesserung der Sehkraft wird dabei weiterhin durch das zwischenzeitliche Entspannen der angespannten und Augenbewegungsmuskeln erreicht, beispielsweise durch Zwinkern oder Blinzeln. Diese Entspannungsübungen werden dem Benutzer visuell oder akustisch vermittelt und sind im Rahmen des Trainingsprogramms eingebunden.

Vorteilhaft wird durch das erfindungsgemäße Trainingssystem die Augenbewegungsmuskulatur gekräftigt, gedehnt, entspannt, aufgebaut und die Durchblutung verbessert.

Insbesondere bei Kindern im Alter von 9 bis 11 Jahren kann durch das erfindungsgemäße Trainingssystem eine Akzeleration und somit eine Entwicklungsbeschleunigung der Augen erreicht werden. Da in dieser Altersspanne die Augäpfel plötzlich schnell anfangen, zu wachsen, aber die Quermuskeln gleichzeitig noch im Wachstum zurückbleiben, drücken die Quermuskeln mit fortschreitendem Wachstum auf das Auge. Dadurch beginnt das Auge, sich nach vorn zu ziehen und eine sogenannte falsche Kurzsichtigkeit entsteht. Diese falsche Kurzsichtigkeit kann durch das Training der Augenbewegungsmuskeln mittels des erfindungsgemäßen Trainingssystems behoben werden. Dadurch werden die Augenbewegungsmuskeln gut entwickelt und trainiert, arbeiten zuverlässig und versorgen so die Augen ausreichend mit Blut, wodurch wiederum der Stoffwechsel in den Augen normal abläuft und die Augen gesund sind.

In einer weiteren bevorzugten Ausführungsform wird das Computerprogrammprodukt des erfindungsgemäßen Trainingssystems zur Stimulation der Augenbewegung sowie zum selbstständigen Trainieren der Augenbewegungsmuskulatur verwendet.

Die Erfindung ist nicht auf die dargestellten und beschriebenen Ausführungsformen beschränkt, sondern umfasst auch alle im Sinne der Erfindung gleich wirkenden Ausführungsformen. Ferner ist es für die Realisierung der Erfindung auch zweckmäßig, die vorbeschriebenen erfindungsgemäßen Ausgestaltungen, Ausführungsformen und Merkmale der Ansprüche in zweckmäßiger Anordnung miteinander zu kombinieren.

### Ausführungsbeispiel

Nachfolgend soll die Erfindung anhand eines Ausführungsbeispiels eingehender erläutert werden. Das Ausführungsbeispiel bezieht sich auf ein Trainingssystem zur Stimulation der Augenbewegung sowie zum selbstständigen Trainieren der Augenbewegungsmuskulatur und soll dabei die Erfindung beschreiben ohne diese zu beschränken.

Anhand von Zeichnungen wird die Erfindung näher erläutert. Dabei zeigt
- Fig. 1: das erfindungsgemäße Trainingssystem mit in Richtung Benutzer gekrümmten Anzeigevorrichtung und integrierter Datenverarbeitungseinrichtung,
- Fig. 2: das erfindungsgemäße Trainingssystem mit flacher Anzeigevorrichtung und integrierter Datenverarbeitungseinrichtung,
- Fig. 3: eine extern angeordnete Datenverarbeitungseinrichtung des erfindungsgemäßen Trainingssystems.

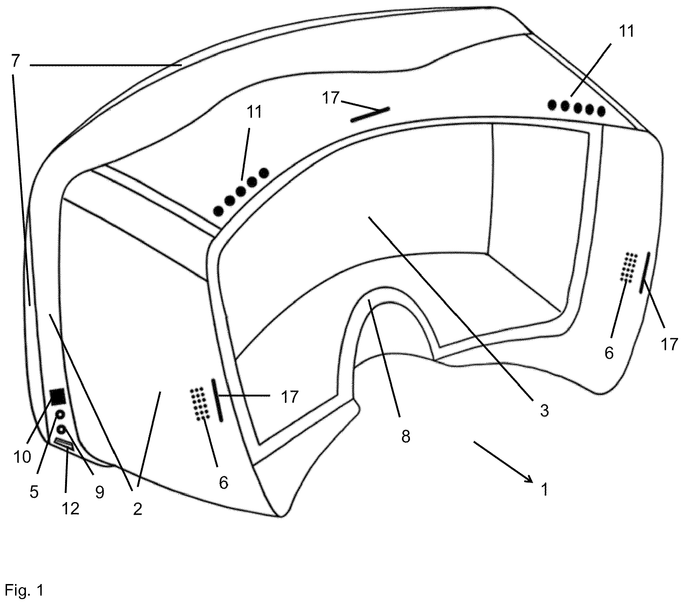

Figur 1 zeigt das erfindungsgemäße Trainingssystem (1), welches ein als Brille ausgebildetes Gehäuse (2) aufweist. Das Gehäuse (2) weist eine Polsterung (8) auf, welche auf dem Gesicht des Benutzers aufliegt. Im Gehäuse (2) integriert ist eine als gekrümmtes Display ausgebildete Anzeigeeinrichtung (3), auf welche der Benutzer beim Aufsetzen des Gehäuses (2) blickt. Das Gehäuse (2) weist einen Lautsprecheranschluss (5) auf, an welchen ein Kopfhörer angeschlossen werden kann. Im Gehäuse integriert ist eine Datenverarbeitungseinrichtung (in Figur 1 nicht gezeigt), welche über u.a. als USB-Anschluss ausgebildete Mittel zur Signalübertragung (12) mit einem externen Ausgabegerät (in Figur 1 nichtgezeigt) gekoppelt ist. Weiterhin weist das Gehäuse (2) eine Stromversorgung (9) sowie einen An-/Ausschalter (10) auf. Durch den An-/Ausschalter (10) wird das Trainingssystem (1) in Betrieb genommen und die elektrischen Komponenten wie Anzeigeeinrichtung (3), Lautsprecher (6) und Datenverarbeitungseinrichtung werden gestartet. Die Energieversorgung erfolgt durch einen im Gehäuse (2) integrierten Energiespeicher (in Figur 1 nicht gezeigt). Über am Gehäuse (2) angebrachte Menütasten (11) kann das Training gesteuert werden, indem beispielsweise ein Trainingsplan eines bestimmten Schwierigkeitsgrades ausgewählt wird. Im Gehäuse (2) sind weiterhin die Halterungen (17) für die Haltevorrichtung angeordnet. Eine Abdeckung (7) schützt die Anzeigeeinrichtung (3) von außen.

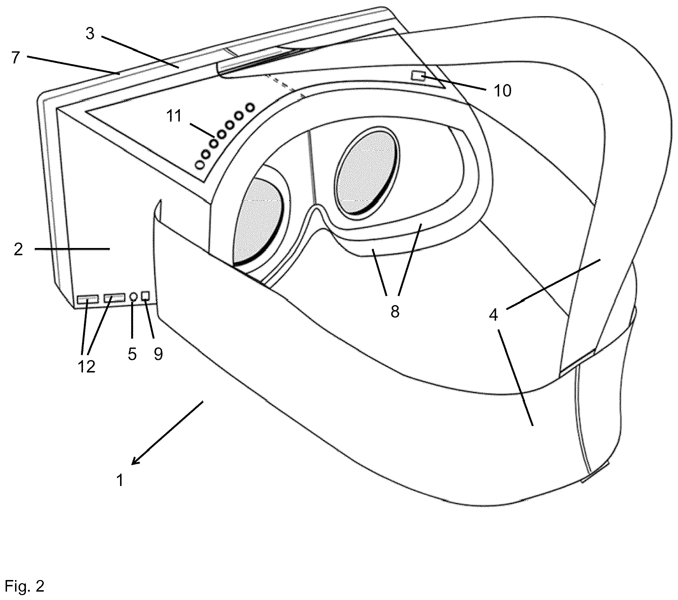

Figur 2 zeigt das erfindungsgemäße Trainingssystem (1), welches ein als VR-Brille ausgebildetes Gehäuse (2) aufweist. Das Gehäuse (2) weist eine Polsterung (8) auf, welche auf dem Gesicht des Benutzers aufliegt. Im Gehäuse (2) integriert ist eine als flaches Display ausgebildete Anzeigeeinrichtung (3), auf welche der Benutzer beim Aufsetzen des Gehäuses (2) blickt. Das Gehäuse (2) weist einen Lautsprecheranschluss (5) auf, an welchen ein Kopfhörer angeschlossen werden kann. Im Gehäuse integriert ist eine Datenverarbeitungseinrichtung (in Figur 2 nicht gezeigt), welche über ua. zwei als USB-Anschlüsse ausgebildete Mittel zur Signalübertragung (12) mit einem externen Ausgabegerät (in Figur 1 nichtgezeigt) gekoppelt ist. Weiterhin weist das Gehäuse (2) eine Stromversorgung (9) sowie einen An-/Ausschalter (10) auf. Durch den An-/Ausschalter (10) wird das Trainingssystem (1) in Betrieb genommen und die elektrischen Komponenten wie Anzeigeeinrichtung (3), Lautsprecher und Datenverarbeitungseinrichtung werden gestartet. Die Energieversorgung erfolgt durch einen im Gehäuse (2) integrierten Energiespeicher (in Figur 2 nicht gezeigt). Über am Gehäuse (2) angebrachte Menütasten (11) kann das Training gesteuert werden, indem beispielsweise ein Trainingsplan eines bestimmten Schwierigkeitsgrades ausgewählt wird. Das Gehäuse (2) weist einen Lautsprecheranschluss (5) auf, an welchen ein Kopfhörer angeschlossen werden kann. Über die im Gehäuse (2) angeordnete, als variables Gurtband ausgebildete Haltevorrichtung (4), kann das Trainingssystem (1) bequem auf den Kopf des Benutzers gesetzt werden. Eine Abdeckung (7) schützt die Anzeigeeinrichtung (3) von außen.

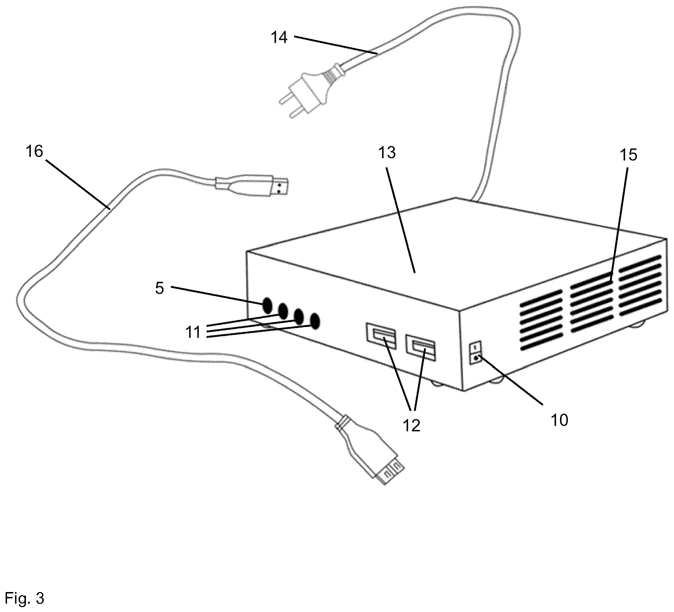

Figur 3 zeigt die extern angeordnete Datenverarbeitungseinrichtung (13) des erfindungsgemäßen Trainingssystems. Die Datenverarbeitungseinrichtung (13) ist dabei nicht im Gehäuse des Trainingssystem angeordnet, wodurch Gewicht und Kompaktheit des Gehäuses reduziert werden. Die Datenverarbeitungseinrichtung (13) weist als USB-Anschlüsse ausgebildete Mittel zur Signalübertragung (12) sowie ein als Verbindungskabel ausgebildetes Mittel zur Signalübertragung (16) auf, durch welche sie mit einem ebenfalls extern angeordneten Ausgabegerät kommunizieren kann. Über einen An-/Ausschalter (10) wird das Trainingssystem ein- und ausgeschaltet. Weiterhin weist die Datenverarbeitungseinrichtung (13) einen Netzstecker (14) zur Stromversorgung auf. Eine Belüftung (15) sorgt dafür, dass die Datenverarbeitungseinrichtung (13) nicht überhitzt wird. Die Datenverarbeitungseinrichtung (13) weist einen Lautsprecheranschluss (5) auf, an welchen ein Kopfhörer angeschlossen werden kann. Über an der Datenverarbeitungseinrichtung (13) angebrachte Menütasten (11) kann das Training gesteuert werden, indem beispielsweise ein Trainingsplan eines bestimmten Schwierigkeitsgrades ausgewählt wird.

Im Folgenden wird exemplarisch der Ablauf von 13 Trainingseinheiten des Trainingsplans in Schwierigkeitsgrad 1 beschrieben. Das Training nach Schwierigkeitsgrad 1 umfasst 13 Trainingseinheiten mit einer Gesamtdauer von ca. 4 min. Indem Schwierigkeitsgrad 1 mindestens 3mal täglich trainiert werden sollte, ergibt sich somit eine Gesamttrainingsdauer pro Tag von ca. 12 min. Im Idealfall ist das Training nach Schwierigkeitsgrad 1 über mindestens einen Tag zu trainieren, aber je nach Bedarf auch länger. Dabei werden vorliegend beide Augen und deren Augenbewegungsmuskulatur ganzheitlich trainiert.

Dabei fokussieren die Augen des Benutzers jeweils auf die von der Anzeigeeinrichtung angezeigten Objekte, welche in Abhängigkeit von der Trainingseinheit variiert werden. Die Bewegung der dargestellten Objekte entspricht der Bewegung der Augen.
- Trainingseinheit 1:
   ∘ 4-maliger Bewegungsablauf der Augen von der Mitte links zur Mitte rechts
   ∘ 2 Sekunden zwinkern (Entspannung der Augen)
- Trainingseinheit 2:
   ∘ 4-maliger Bewegungsablauf der Augen von der Mitte oben zur Mitte unten
   ∘ 2 Sekunden zwinkern (Entspannung der Augen)
- Trainingseinheit 3:
   ∘ 4-maliger Bewegungsablauf der Augen von diagonal links unten nach diagonal rechts oben
   ∘ 2 Sekunden zwinkern (Entspannung der Augen)
- Trainingseinheit 4:
   ∘ 4-maliger Bewegungsablauf der Augen von diagonal rechts unten nach diagonal links oben
   ∘ 2 Sekunden zwinkern (Entspannung der Augen)
- Augen für 20 Sekunden schließen und entspannen
- Trainingseinheit 5:
   ∘ 4-maliger Bewegungsablauf der Augen von links oben nach rechts oben nach rechts unten nach links unten
   ∘ 2 Sekunden zwinkern (Entspannung der Augen)
- Trainingseinheit 6:
   ∘ 4-maliger Bewegungsablauf der Augen von rechts oben nach links oben nach links unten nach rechts unten
   ∘ 2 Sekunden zwinkern (Entspannung der Augen)
- Trainingseinheit 7:
   ∘ 2-maliger Bewegungsablauf der Augen im Uhrzeigersinn, beginnend bei 12:00, über 01:00, 02:00, 03:00, 04:00, 05:00, 06:00, 07:00, 08:00. 09:00, 10:00, 11:00 und wieder endend bei 12:00
   ∘ 2 Sekunden zwinkern (Entspannung der Augen)
- Trainingseinheit 8:
   ∘ 2-maliger Bewegungsablauf der Augen entgegengesetzt zum Uhrzeigersinn, beginnend bei 12:00, über 09:00, 06:00, 03:00 und wieder endend bei 12:00
   ∘ 2 Sekunden zwinkern (Entspannung der Augen)
- Augen für 30 Sekunden schließen und entspannen
- Trainingseinheit 9:
   ∘ 2-maliger Bewegungsablauf der Augen entgegengesetzt zum Uhrzeigersinn, beginnend bei 12:00, über 11:00, 10:00, 09:00, 08:00, 07:00, 06:00, 05:00, 04:00, 03:00, 02:00, 01:00 und wieder endend bei 12:00
   ∘ 2 Sekunden zwinkern (Entspannung der Augen)
- Trainingseinheit 10:
   ∘ 2-maliger Bewegungsablauf der Augen im Uhrzeigersinn, beginnend bei 12:00, über 03:00, 06:00, 09:00 und wieder endend bei 12:00
   ∘ 2 Sekunden zwinkern (Entspannung der Augen)
- Trainingseinheit 11:
   ∘ 2-maliger Bewegungsablauf der Augen kreisend im Uhrzeigersinn für jeweils 15 Sekunden
   ∘ 2 Sekunden zwinkern (Entspannung der Augen)
- Trainingseinheit 12:
   ∘ 2-maliger Bewegungsablauf der Augen kreisend entgegen dem Uhrzeigersinn für jeweils 15 Sekunden
   ∘ 2 Sekunden zwinkern (Entspannung der Augen)
- Trainingseinheit 13:
   ∘ Einmalig für ca. 8 Sekunden Augen schließen, Augenmuskeln fest anspannen und Augen rollen

### Bezugszeichen

- 1: Trainingssystem
- 2: Gehäuse
- 3: Anzeigeeinrichtung
- 4: Haltevorrichtung
- 5: Lautsprecheranschluss
- 6: Lautsprecher
- 7: Abdeckung
- 8: Polsterung
- 9: Stromversorgung
- 10: An-/Ausschalter
- 11: Menütasten
- 12: Mittel zur Signalübertragung (USB-Anschluss)
- 13: Datenverarbeitungseinrichtung
- 14: Netzstecker
- 15: Belüftung
- 16: Mittel zur Signalübertragung (Verbindungskabel)
- 17: Halterung für die Haltevorrichtung

## Patentansprüche

1. Trainingssystem zur Stimulation der Augenbewegung sowie zum selbstständigen Trainieren der Augenbewegungsmuskulatur, aufweisend
∘ elektrische Komponenten, welche voneinander beabstandet ausgebildet sind, umfassend
▪ mindestens eine Datenverarbeitungseinrichtung,
▪ mindestens eine Anzeigeeinrichtung zur optischen Signalisierung von veränderlich ausgebildeten Objekten, und
▪ mindestens einen Lautsprecher,
wobei die elektrischen Komponenten durch Mittel zur Signalübertragung lösbar miteinander verbunden sind, sowie
∘ ein Gehäuse, welches am Kopf anordbar ausgebildet ist, und
∘ eine Haltevorrichtung, welche mit dem Gehäuse lösbar verbunden ist,
wobei die elektrischen Komponenten zumindest teilweise im oder am Gehäuse angeordnet sind,
wobei die mindestens eine Anzeigeeinrichtung vor den Augen eines Benutzers angeordnet und derart ausgebildet ist, mindestens ein Objekt in allen Raumrichtungen starr oder beweglich darzustellen, um das Blickfeld des Benutzers zu bedienen.

2. Trainingssystem nach Anspruch 1, **dadurch gekennzeichnet, dass** die mindestens eine Anzeigeeinrichtung als Display ausgebildet ist.

3. Trainingssystem nach Anspruch 1 oder 2, **dadurch gekennzeichnet, dass** das Gehäuse als Brille oder Maske ausgebildet ist.

4. Trainingssystem nach einem der Ansprüche 1 bis 3, **dadurch gekennzeichnet, dass** die Haltevorrichtung als verstellbares Band, Gurtband oder Gurt ausgebildet ist.

5. Trainingssystem nach einem der Ansprüche 1 bis 4, weiterhin umfassend mindestens ein Fokussierrad, welches am Gehäuse angeordnet ist.

6. Trainingssystem nach einem der Ansprüche 1 bis 5, **dadurch gekennzeichnet, dass** die mindestens eine Datenverarbeitungseinrichtung extern vom Trainingssystem angeordnet ist.

7. Trainingssystem nach einem der Ansprüche 1 bis 6, **dadurch gekennzeichnet, dass** die mindestens eine Datenverarbeitungseinrichtung über ein Modul zur Datenübertragung mit einem externen Ausgabegerät gekoppelt ist.

8. Trainingssystem nach einem der Ansprüche 1 bis 7, weiterhin umfassend mindestens einen Energiespeicher.

9. Trainingssystem nach einem der Ansprüche 1 bis 8 zur Verwendung in einem Verfahren zur Stimulation der Augenbewegung sowie zum selbstständigen Trainieren der Augenbewegungsmuskulatur, wobei das Verfahren umfasst:
∘ das Anordnen des Gehäuses durch die Haltevorrichtung am Kopf des Benutzers, sodass die mindestens eine Anzeigeeinrichtung vor den Augen und der mindestens eine Lautsprecher vor mindestens einem Ohr des Benutzers angeordnet ist, und anschließend
∘ die Darstellung von Objekten in allen Raumrichtungen, auf welche sich der Benutzer fokussiert und mindestens ein Auge am Rande des Blickfelds bewegt, und
∘ die akustische Wiedergabe von Geräuschen, auf welche der Benutzer reagiert,
wobei durch die Datenverarbeitungseinrichtung und/oder durch das externe Ausgabegerät ein szenarioabhängiges Steuersignal über die Mittel zur Signalübertragung an die mindestens eine Anzeigeeinrichtung und/oder den mindestens einen Lautsprecher übermittelt wird, wodurch der Trainingsablauf vorgegeben oder selbstständig angepasst wird.

10. Trainingssystem nach einem der Ansprüche 1 bis 8 zur Verwendung in einem Verfahren zur Stimulation der Augenbewegung sowie zum selbstständigen Trainieren der Augenbewegungsmuskulatur, wobei das Verfahren weiterhin umfasst, dass
∘ über mindestens einen Sensor die Bewegung der Augen des Benutzers als Daten erfasst werden und die erfassten Daten über Mittel zur Signalübertragung an die mindestens eine Datenverarbeitungseinrichtung übermittelt werden, und
∘ die Daten von der Datenverarbeitungseinrichtung an ein externes Ausgabegerät übermittelt werden, wobei
∘ die Daten durch die mindestens eine Datenverarbeitungseinrichtung und/oder das externe Ausgabegerät verarbeitet und ausgewertet werden
∘ und darauf basierend ein szenarioabhängiges Steuersignal über die Mittel zur Signalübertragung an die mindestens eine Anzeigeeinrichtung und/oder den mindestens einen Lautsprecher übermittelt wird, wodurch das Training selbstständig angepasst wird.

11. Computerprogrammprodukt, wobei das Computerprogrammprodukt eine erste Teilkomponente aufweist, die als visuelle Trainingsanleitung dient, und eine zweite Teilkomponente aufweist, die als akustische Trainingsanleitung dient, wobei die erste und die zweite Teilkomponente informationstechnisch miteinander gekoppelt sind.

12. Datenverarbeitungseinrichtung und/oder externes Ausgabegerät, auf der das Computerprogrammprodukt nach Anspruch 11 gespeichert ist, wobei die erste Teilkomponente des Computerprogrammprodukts auf der Datenverarbeitungseinrichtung oder dem externen Ausgabegerät und die zweite Teilkomponente des Computerprogrammprodukts auf der Datenverarbeitungseinrichtung oder auf einem externen Ausgabegerät gespeichert ist.

13. Verwendung eines Trainingssystems wie in einem der Ansprüche 1 bis 8 definiert, zur Stimulation der Augenbewegung sowie zum selbstständigen Trainieren der Augenbewegungsmuskulatur.

14. Verwendung eines Trainingssystems wie in einem der Ansprüche 1 bis 8 definiert, zur gezielten Verbesserung der Sehkraft oder zur Kräftigung und Stärkung des Sehvermögens.

15. Verwendung eines Computerprogrammproduktes nach Anspruch 11 zur Stimulation der Augenbewegung sowie zum selbstständigen Trainieren der Augenbewegungsmuskulatur.
